# EUROPÄISCHE PATENTSCHRIFT

(11) **EP 3 261 687 B1**
(45) Veröffentlichungstag und Bekanntmachung des Hinweises auf die Patenterteilung: **29.03.2023**
(21) Anmeldenummer: 15804499.0
(22) Anmeldetag: 03.12.2015
(51) Int. Cl.: A61L 31/08, A61L 31/14

(54) **IMPLANTAT ZUM ÜBERDECKEN VON KNOCHENDEFEKTEN IM KIEFERBEREICH SOWIE VERFAHREN ZU DESSEN HERSTELLUNG**
IMPLANT FOR COVERING MAXILLARY BONE DEFECTS AND METHOD FOR PRODUCING THE SAME
IMPLANT POUR MASQUER DES DÉFAUTS OSSEUX DANS LA RÉGION MAXILLAIRE ET PROCÉDÉ POUR LE PRODUIRE

(30) Priorität: 24.02.2015 DE 102015102597
(43) Veröffentlichungstag der Anmeldung: 03.01.2018
(73) Patentinhaber: Botiss Biomaterials GmbH, 15806 Zossen (DE)
(72) Erfinder: TADIC, Drazen, 14129 Berlin (DE); BIELENSTEIN, Oliver, 10629 Berlin (DE)
(74) Vertreter: Friderichs, Gunther
(86) Internationale Anmeldenummer: PCT/EP2015/078499
(87) Internationale Veröffentlichungsnummer: WO 2016/134797

(56) Entgegenhaltungen:
- WO-A2-2015/185597
- DE-A1-102006 011 348
- JP-A- 2010 082 146
- JP-A- 2011 136 967
- US-A1- 2013 304 134
- IAN JOHNSON ET AL: "A Study on Factors Affecting the Degradation of Magnesium and a Magnesium-Yttrium Alloy for Biomedical Applications", PLOS ONE, vol. 8, no. 6, 14 June 2013 (2013-06-14), page e65603, XP055332048, DOI: 10.1371/journal.pone.0065603

## Beschreibung

### Gebiet der Erfindung

Die Erfindung betrifft ein bioresorbierbares Implantat zum Überdecken von Knochendefekten im Kieferbereich sowie ein Verfahren zu dessen Herstellung. Insbesondere betrifft die Erfindung ein Implantat, welches über eine mit einem Knochenersatzmaterial gefüllte Defektstelle gesetzt wird und über welchem sodann das Weichgewebe verschlossen wird.

### Hintergrund der Erfindung

Implantate zum Abdecken von Knochendefekten im Kieferbereich sind bekannt. Es gibt sowohl bioresorbierbare Implantate, welche sich nach dem Einsetzen langsam auflösen als auch Implantate, die entweder im Körper verbleiben oder nach einer gewissen Zeit wieder entfernt werden müssen.

Einen generellen Überblick über bekannte Implantate zum Abdecken von Knochendefekten liefert der Artikel Liu at al "Mechanisms of Guided Bone Regeneration: A Review, The Open Dentistry Journal, 2014, 8, P. 56 - 65".

So gibt es nicht resorbierbare PTFE-Membranen. Diese lassen sich leicht einsetzen, müssen aber explantiert werden.

Weiter gibt es als resorbierbares Material insbesondere Collagenmembranen.

Diese haben den Vorteil, dass sie nicht explantiert werden müssen, was allerdings wiederum mit dem Nachteil verbunden ist, dass sie nicht besonders dicht sind, so dass die Gefahr besteht, dass Weichgewebe in die gefüllte Knochendefektstelle einwächst oder Knochenmaterial aus der Defektstelle austritt.

Das Dokument JP 2010 082146 zeigt eine poröse Membran, welche mit einer Magnesiumschicht als Stützschicht versehen ist. Die Dokumente US 2013/0304134 A1 sowie DE 10 2006 011 348 A1 zeigen biodegradierbare Magnesiumimplantate, welche mit einer passivierten Schutzschicht versehen sind.

IAN JOHNSON ET AL: "A Study on Factors Affecting the Degradation of Magnesium and a Magnesium-Yttrium Alloy for Biomedical Applications", PLOS ONE, Bd. 8, Nr. 6, 14. Juni 2013 (2013-06-14), Seite e65603, XP055332048, DOI: 10.1371/journal.pone.0065603 ist eine Studie zu den Degerationseigenschaften von Magnesium und verschiedenen Magnesiumlegierungen.

Das Dokument JP 2011 136967 A zeigt ein Implantat, welches als Gitter aus einer Magnesiumlegierung ausgebildet ist.

### Aufgabe der Erfindung

Der Erfindung liegt demgegenüber die Aufgabe zugrunde, die genannten Nachteile des Standes der Technik zu reduzieren.

Es ist insbesondere eine Aufgabe der Erfindung, ein bioresorbierbares Implantat bereitzustellen, welches einfach eingesetzt werden kann und Weichgewebe vom Knochenmaterial, insbesondere vom Knochenersatzmaterial, in der Anfangsphase nach dem Einsetzen zuverlässig trennt.

### Zusammenfassung der Erfindung

Die Aufgabe der Erfindung wird bereits durch ein Implantat zum Überdecken von Knochendefekten im Kieferbereich nach Anspruch 1 gelöst.

Bevorzugte Ausführungsformen und Weiterbildungen der Erfindung sind dem Gegenstand der Unteransprüche zu entnehmen.

Die Erfindung betrifft ein Implantat zum Überdecken von Knochendefekten im Kieferbereich, gemäß den Ansprüchen welches aus einer Magnesiumfolie besteht.

Unter einer Magnesiumfolie wird eine Folie aus Magnesium oder einer Magnesiumlegierung verstanden, welche überwiegend, also zu mehr als 50 % (%-Angaben, soweit nicht anders angegeben immer in Gewichts%), aus Magnesium besteht.

Es hat sich in überraschender Weise gezeigt, dass eine derartige Magnesiumfolie zu einer sauberen Trennung des Weichgewebes von der Defektstelle führt, welche insbesondere mit einem Knochenersatzmaterial, wie insbesondere auch homologen oder autologen Knochentransplantaten aufgefüllt wird.

Die bioresorbierbaren Eigenschaften von Magnesium sind als solche bekannt. Es ist aber durchaus überraschend, dass bereits eine Folie im Kieferbereich derart lange einem korrosiven Angriff widersteht, dass bis dahin das Wachstum von natürlichem Knochengewebe in die Defektstelle derart abgeschlossen ist, dass es bis zum zumindest teilweisen Abbau des Implantats nicht zum Einwachsen von Weichgewebe kommt.

Die Erfindung eignet sich für alle Arten der Behandlung von Knochendefekten im Kieferbereich, insbesondere auch von Sinuslift-Korrekturen.

Als Knochenersatzmaterial werden insbesondere kalziumphosphat- oder hydroxylapatithaltige Materialien verwendet. Es kann sich dabei sowohl um künstliche als auch um natürliche Materialien handeln, insbesondere auch um Materialien, welche aus Spenderknochen, beispielsweise humanen oder porcinen Ursprungs, hergestellt sind. Das Knochenersatzmaterial kann sowohl als Granulat als auch als angepasster Formkörper, welcher zumindest abschnittweise der Kontur der Defektstelle angenähert ist, eingesetzt werden.

Das Implantat ist vorzugsweise formstabil. Dies bedeutet, dass beispielsweise ein gebogenes Implantat nicht aufgrund seiner eigenen Gewichtskraft die Form ändert.

Hierfür wird gemäß der Erfindung eine Magnesiumfolie mit einer Dicke zwischen 70 und 200 µm verwendet.

Vorzugsweise ist das Implantat gekrümmt ausgebildet. So kann es auf die Defektstelle aufgesetzt und im günstigsten Falle sogar auf dem Kieferkamm verklemmt werden.

Es ist insbesondere zumindest abschnittsweise ein Krümmungsradius von 0,5 bis 10 cm, vorzugsweise von 0,7 bis 1,5 cm, vorgesehen.

Es hat sich gezeigt, dass sich eine Magnesiumfolie der genannten Dicke insbesondere auch in einem Winkel > 30° biegen lässt. Der Winkel wird definiert durch den Winkel, welchen die Tangenten der Enden des Implantats zueinander einnehmen. Es ist insbesondere vorgesehen, dass sich die Seiten des Implantats parallel gegenüberstehen oder sogar einen Winkel von mehr als 180° einnehmen, um das Implantat zu fixieren, insbesondere aufzuklemmen.

Bei einer Weiterbildung der Erfindung weist das Implantat zumindest eine Aussparung für einen Zahn auf.

Das Implantat ist vorzugsweise an einem vorderen und/oder hinteren Ende derart ausgespart, dass die angrenzenden Seiten den Zahn teilweise umschließen. Hierdurch wird zum einen das Einsetzen erleichtert, zum anderen wird die Gefahr des Einwachsens von Weichgewebe weiter reduziert.

Das Implantat hat vorzugsweise eine Größe von 0,5 bis 25 cm². Sofern eine Aussparung für einen Zahn vorgesehen ist, nimmt diese vorzugsweise eine Fläche von mehr als 0,25 cm² ein.

Bei einer Weiterbildung der Erfindung umfasst das Implantat eine beschichtete und/oder geätzte Oberfläche.

Es hat sich herausgestellt, dass insbesondere ein in eine Säure eingetauchtes Implantat hinsichtlich möglicher Biegeradien sowie hinsichtlich Korrosionseigenschaften verbesserte Eigenschaften aufweist.

Das Implantat hat vorzugsweise ferner eine glatte Oberfläche mit einem Mittenrauwert Ra von unter 0,08, vorzugsweise von unter 0,03 und besonders bevorzugt von unter 0,02 µm. Insbesondere durch eine Säurebehandlung beispielsweise mit Salpetersäure lässt sich eine derart glatte Oberfläche erreichen.

Als Oberflächenbehandlung ist insbesondere ein Ätzen und/oder Passivieren vorgesehen. Als besonders geeignet hat sich erwiesen, ein vorgereinigtes Implantat, insbesondere ein mit Säure vorgereinigtes Implantat, für mindestens 10 Minuten in Flusssäure einzutauchen, wodurch sich durch agitierte Immersion eine Schutzschicht aus Magnesiumfluorid bildet. Die passivierte Oberfläche, insbesondere die Magnesiumfluorid-Schicht hat vorzugsweise eine Dicke von weniger als 5, besonders bevorzugt von weniger als 2 µm.

Insbesondere ist vorgesehen, eine Schicht zu erzeugen, deren Dicke zwischen 0,2 und 2 µm, bevorzugt zwischen 0,7 und 1,5 µm, liegt.

Es bildet sich so eine gut verzahnte, vorzugsweise Porenfreie, kratzfeste Schicht, welche auch bei engen Biegeradien nicht zum Einreißen neigt.

Die Folie weist erfindungsgemäß zumindest in einem mittleren Bereich, also insbesondere dem Bereich, der direkt über der Defektstelle angeordnet ist, keine Öffnungen auf. So wird ein dichtes Verschließen des Defekts sichergestellt.

Bei einer Weiterbildung der Erfindung ist das Implantat abschnittsweise, insbesondere randseitig, strukturiert und/oder perforiert ausgebildet. Insbesondere über eine Perforation, beispielsweise in Form einer Gitterstruktur, oder über eine Strukturierung, insbesondere eine Plissierung, kann das Biegen des Implantats erleichtert und/oder dessen Anhaftung zum angrenzenden Gewebe verbessert werden.

Das Implantat besteht vorzugsweise aus reinem Magnesium (Reinheitsgrad über 99 %) oder einer Magnesiumlegierung. Diese kann 1 bis 6 % Yttrium, 0,5 bis 3 % Zink, 0,1 bis 2 % Kalzium und/oder 0,6 bis 1,5 % Mangan umfassen.

Die Magnesiumfolie weist dagegen vorzugsweise weniger als 500 ppm Eisen, Kupfer und/oder Nickel auf.

Die Erfindung betrifft des Weiteren ein Set zum Überdecken von Knochendefekten im Kieferbereich, welches ein vorstehend beschriebenes Implantat sowie Pins zur Fixierung des Implantats im Kiefer umfasst. Unter Pins im Sinne der Erfindung werden insbesondere Stifte, Nägel oder Schrauben verstanden. Auch die Pins bestehen vorzugsweise aus Magnesium bzw. einer Magnesiumlegierung.

Das Implantat kann, wie es bei einer Weiterbildung der Erfindung vorgesehen ist, Löcher aufweisen, durch welche die Pins geführt werden. Denkbar ist aber auch, mit dem Pin selber das Befestigungsloch einzubringen, insbesondere wenn dieser eine Spitze aufweist.

Das erfindungsgemäße Implantat kann wie folgt hergestellt werden.

Zunächst wird eine Magnesiumfolie bereitgestellt. Die Magnesiumfolie wird auf die Länge einer zu überdeckenden Stelle gekürzt und die Magnesiumfolie wird gebogen.

Das Biegen der Magnesiumfolie erfolgt vorzugsweise mittels einer Form oder mittels einer Biegemaschine, da so die Gefahr von Knicken und Kanten reduziert wird.

Um das Implantat auf die gewünschte Länge zu kürzen und/oder um Aussparungen etwa für einen Zahn einzubringen, kann dieses beispielsweise geschnitten oder ausgestanzt werden.

Die Magnesiumfolie wird zum Ausbilden einer Schicht vorzugsweise mit Flusssäure behandelt, insbesondere über einen Zeitraum von mehr als 10, vorzugsweise mehr als 12 Stunden, und insbesondere mit einer mehr als 30 %igen Flusssäure.

Das Implantat kann für ein Verfahren zur kosmetischen und/oder chirurgischen Kieferrekonstruktion verwendet werden, wobei das vorstehend beschriebene Implantat über einer Defektstelle, welche insbesondere mit einem Knochenersatzmaterial aufgefüllt ist, platziert wird und das Weichgewebe über der Defektstelle geschlossen wird, insbesondere durch Vernähen.

Es hat sich gezeigt, dass es mit der erfindungsgemäßen Magnesiumfolie auf einfache Weise möglich ist, ein bioresorbierbares Implantat bereitzustellen, welches während der Bildung natürlichen Knochenmaterials ein Einwachsen von Weichgewebe weitgehend verhindert.

Das Implantat braucht nicht entnommen zu werden, sondern baut sich ab. Überraschenderweise kommt es trotz der verhältnismäßig großen Oberfläche der verwendeten Folie nicht in unerwünscht hohem Maße zu blasenbildenden Gasentwicklungen.

Nach der Bildung natürlichen Knochengewebes kann sodann beispielsweise ein Stiftzahnimplantat in die ehemalige Defektstelle eingesetzt werden. Es ist möglich, das Stiftzahnimplantat bereits einzusetzen, bevor sich die Magnesiumfolie abgebaut hat.

Das erfindungsgemäße Implantat eignet sich insbesondere für folgende Anwendungsmöglichkeiten:
Zum einen können als Magnesiumfolie ausgebildete Implantate zur Behandlung von Verletzungen der Schneiderschen Membran verwendet werden.

Die Schneidersche Membran trennt den Kieferknochen von der Kieferhöhle. Bei Verletzung derselbigen besteht extreme Infektionsgefahr.

Es hat sich herausgestellt, dass durch ein Einsetzen einer dünnen Magnesiumfolie eine derartige Defektstelle verschlossen werden kann. Das Magnesium scheint dabei vermutlich der Erhöhung des pH-Wertes auch eine entzündungshemmende Wirkung zu haben und beschleunigt des Weiteren die Bildung von natürlichem Knochengewebe, etwa beim Einsetzen von Knochenfüllmaterial.

Eine weitere Verwendungsmöglichkeit ist die Vermeidung von Wunddehiszenzen, welche bei herkömmlichen Membranen, etwa bei PTFE-Membranen, häufig vorkommen.

Diese Materialien müssen auch in einer weiteren Operation entfernt werden.

Es hat sich herausgestellt, dass Nahtdehiszenzen über einer Magnesiumfolie von selbst zu einem vollständigen Wundverschluss führen.

Es können so Defekte von bis zu 10 mm toleriert werden, ohne dass es zu Infektionen kommt.

Erstaunlicherweise korrodiert die Folie recht langsam und bleibt über einen langen Zeitraum mechanisch stabil.

Weiter können Defektstellen im Kieferkamm behoben werden.

Insbesondere kann eine Magnesiumfolie unter einem Periostlappen selbstständig eine Regeneration der kortikalen Wand herbeiführen. Die Behandlung zur Regeneration der kortikalen Wand ist in jedem Bereich derselben möglich, insbesondere der lateralen kortikalen Wand.

Es bildet sich unter Defektstellen wie beispielsweise dem freiliegenden Bereich eines Stiftimplantats eine kortikale Wand um die Defektstelle herum.

Schließlich können Magnesiumfolien auch präventiv zum initialen postoperativen Infektionsschutz verwendet werden.

Weiter eignen sich die erfindungsgemäßen Implantate aufgrund der Formstabilität zum Formen von komplexen, dreidimensionalen Knochenregeneraten.

Dabei wird das Füllmaterial unterhalb der Magnesiumfolie in die Defektstelle eingebracht.

Die Magnesiumfolie sichert aufgrund der dreidimensionalen, formstabilen Freiformfläche diese Form bis zur knöchernen Konsolidierung und löst sich sodann vollständig auf.

### Kurzbeschreibung der Zeichnungen

Der Gegenstand der Erfindung soll im Folgenden bezugnehmend auf Ausführungsbeispiele anhand der Zeichnungen Fig. 1 bis Fig. 8 näher erläutert werden.
Fig. 1 zeigt ein Ausführungsbeispiel eines Implantats 1 zum Überdecken von Knochendefekten im Kieferbereich.
Fig. 2 zeigt das Implantat in einer Seitenansicht.
Fig. 3 zeigt eine weitere Ausführungsform eines Implantats.
Fig. 4 bis Fig. 6 zeigen rasterelektronenmikroskopische Aufnahmen eines Implantats.
Fig. 7 zeigt schematisch die Verwendung eines erfindungsgemäßen Implantats zur Prävention oder Behandlung von Verletzungen der Schneiderschen Membran.
Fig. 8 zeigt schematisch die Verwendung eines erfindungsgemäßen Implantats zur sogenannten "lateralen Augmentation" (Kieferknochen-Rekonstruktion).

### Detaillierte Beschreibung der Zeichnungen.

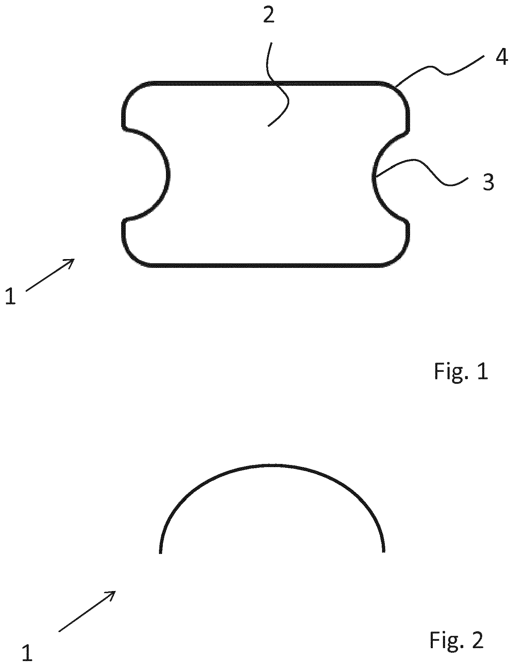

Fig. 1 zeigt ein erstes Ausführungsbeispiel eines Implantats zum Überdecken von Knochendefekten im Kieferbereich.

Das Implantat 1 besteht in diesem Ausführungsbeispiel aus einer 50 bis 150 µm dicken Magnesiumfolie 2, welche insbesondere aus einer Magnesiumlegierung besteht. Das Implantat 1 hat abgerundete Ecken und weist in diesem Ausführungsbeispiel zwei Aussparungen 3 auf, welche für einen angrenzenden Zahn vorgesehen sind.

Das Implantat ist also insbesondere ausgebildet, eine Defektstelle im Kiefer zu überdecken, in welcher ein Zahn oder mehrere Zähne fehlen.

Fig. 2 zeigt das in Fig. 1 dargestellte Implantat 1 in einer Seitenansicht, und zwar in einer Draufsicht auf die Aussparung (3 in Fig. 1).

Zu erkennen ist, dass das Implantat gekrümmt ist. In diesem Ausführungsbeispiel liegen sich die Enden des Implantats fast gegenüber, so dass dieses auf einem Kieferkamm fixiert, insbesondere aufgeklemmt oder aufgeklipst werden kann.

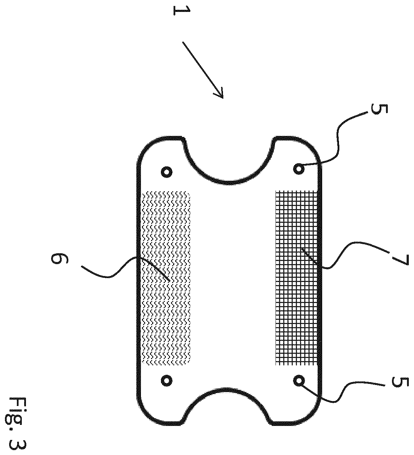

Fig. 3 zeigt eine Weiterbildung eines Implantats 1.

Das Implantat 1 weist zum einen Löcher 5 auf, welche dem Einführen von Fäden, Stiften, Schrauben oder Nägeln dienen.

Weiter umfasst das Implantat 1 in diesem Ausführungsbeispiel randseitig einen netzartigen Bereich 7, also einen Bereich, welcher Aussparungen aufweist. Diese Aussparungen können dem erleichterten Biegen oder dem verbesserten Anhaften am Gewebe dienen.

Weiter dargestellt ist auch ein strukturierter Bereich 6. Dieser kann insbesondere als Plissierung ausgebildet sein und dient der Anpassung des Implantats 1 an dem Kieferknochen.

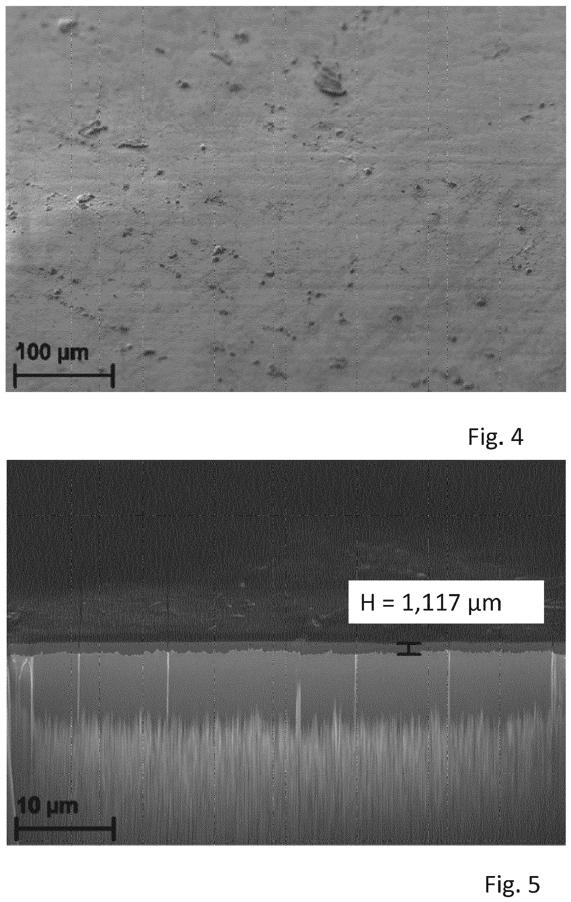

Fig. 4 zeigt eine rasterelektronenmikroskopische Aufnahme der Oberfläche eines Implantats, bei welchem eine Magnesiumfolie mittels Eintauchen in Flusssäure mit einer Magnesiumfluoridbeschichtung versehen wurde.

Es ergibt sich eine etwa 1 µm dünne glatte, kratzfeste Schicht, die auch engere Biegeradien ermöglicht.

Fig. 5 zeigt ebenfalls eine rasterelektronenmikroskopische Aufnahme eines Schnitts der Folie, in welcher die Magnesiumfluoridschicht bereits gut zu erkennen ist.

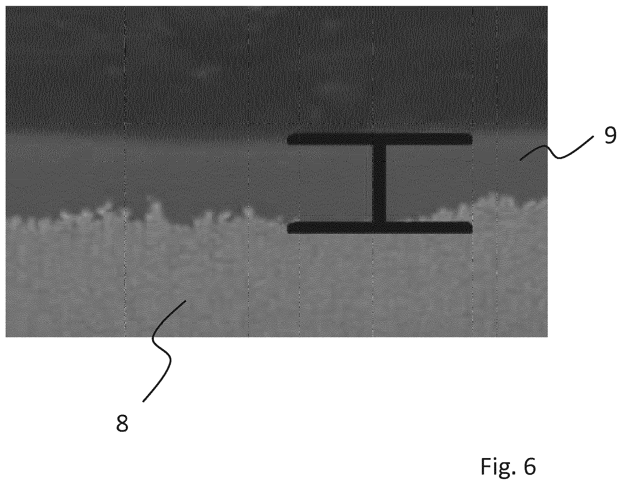

Fig. 6 zeigt eine Detaildarstellung der Fig. 5.

Zu erkennen ist die auf der Magnesiumschicht 8 ausgebildete dünne Magnesiumfluoridschicht 9.

Es ist gut zu sehen, dass die Magnesiumfluoridschicht 9 mit der darunterliegenden Magnesiumschicht gut verzahnt ist, womit vermutlich unter anderem die gute Anhaftung der Schicht zu erklären ist.

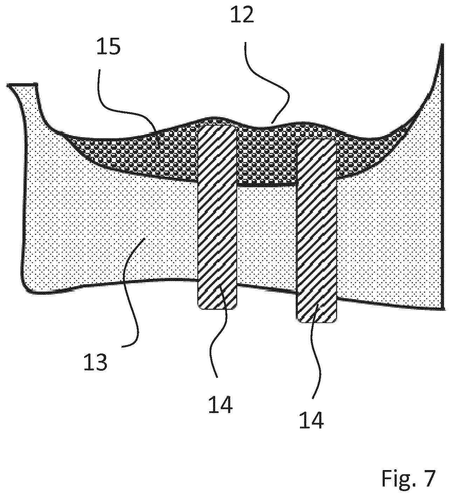

Fig. 7 zeigt in einer schematischen Darstellung eine erste Verwendungsmöglichkeit des erfindungsgemäßen Implantats.

Das als Magnesiumfolie ausgebildete erfindungsgemäße Implantat kann zur Behandlung von verletzten Schneiderschen Membranen verwendet werden.

Insbesondere beim Einsetzen von Stiftimplantaten 14, wie es hier dargestellt ist, die der Aufnahme eines Zahnimplantats dienen, kann es zur Verletzung der Schneiderschen Membran 12 kommen, die den Kieferknochen 13 von der Kieferhöhle trennt.

In vielen Fällen wird, wie dies hier dargestellt ist, ein Zwischenraum zwischen Kieferknochen 13 und Schneiderscher Membran 12 mit einem Knochenersatzmaterial, insbesondere einem Kalziumphosphatgranulat, aufgefüllt, um die Dicke des zurückgebildeten Kieferknochens derart zu erhöhen, dass das Implantat 14 eingesetzt werden kann.

Die Verletzung der Schneiderschen Membran 12 geht mit einer extrem hohen Infektionsgefahr einher. Wird eine derartige Verletzung beim operativen Eingriff bemerkt, so wird in der Regel der Eingriff abgebrochen und erst nach einer Heilungsphase von mehreren Monaten erneut versucht, das Stiftimplantat 14 einzusetzen.

Es hat sich gezeigt, dass dies durch die Verwendung eines erfindungsgemäßen Implantats in Form einer Magnesiumfolie, welche die Schneidersche Membran 12 verschließt, vermieden werden kann.

So kann etwa im Fall eines Defekts die Magnesiumfolie entweder durch die Bohrungen der Stiftimplantate 14 oder durch eine laterale Öffnung des Kieferkamms 13 eingesetzt werden.

Es versteht sich, dass die Magnesiumfolie, wenn diese durch die Bohrungen für die Stiftimplantate eingesetzt wird, vorher beispielsweise zusammengerollt werden kann.

Die Schneidersche Membran 12 ist durch die Magnesiumfolie wieder verschlossen. Erstaunlicherweise sorgt bereits eine dünne Magnesiumfolie von mindestens 50 µm für einen hinreichenden Verschluss, ohne innerhalb einer sehr kurzen Zeitdauer abgebaut zu werden, wie dies eigentlich zu erwarten ist.

Die Magnesiumfolie kann also dazu dienen, dass der Eingriff unmittelbar fortgesetzt werden kann und bietet zudem noch den Vorteil, dass die Magnesiumfolie, insbesondere wenn diese hinreichend dick ausgebildet ist, einen guten Widerhalt für das eingesetzte Knochenmaterial bildet. Das Wachsen von natürlichem Knochengewebe wird durch die Magnesiumfolie ebenfalls begünstigt.

Es versteht sich, dass die Magnesiumfolie aber auch ohne eine Verletzung der Schneiderschen Membran präventiv eingesetzt werden kann.

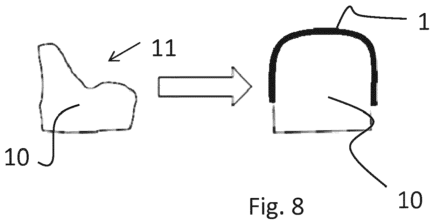

Fig. 8 zeigt schematisch die Verwendung eines erfindungsgemäßen Implantats 1 zur dreidimensionalen Rekonstruktion von Defektstellen im Kieferknochen.

Ein Kieferknochen 10, welcher eine Defektstelle 11 aufweist, wird mit einem gebogenen Implantat 1, bestehend aus einer Magnesiumfolie, überdeckt. Das Implantat 1 bildet dabei eine dreidimensionale Freiformfläche.

Wie auf der rechten Abbildung zu erkennen ist, folgt das sich bildende Knochengewebe des Kieferkamms 10 dieser Freiformfläche und bildet einen abgerundeten Kieferkamm, welcher der natürlichen Form nahe kommt. Diese Annäherung ist insbesondere in der Regel besser als bei Verwendung von eingesetzten Knochenblöcken aus Spendermaterial.

Durch die Erfindung konnte auf einfache Weise ein resorbierbares Implantat zum Überdecken von Knochendefekten im Kieferbereich bereitgestellt werden.

### Bezugszeichenliste

- 1: Implantat
- 2: Magnesiumfolie
- 3: Aussparung
- 4: Ecke
- 5: Loch
- 6: strukturierter Bereich
- 7: netzartiger Bereich
- 8: Magnesiumschicht
- 9: Magnesiumfluoridschicht
- 10: Kieferkamm
- 11: Defektstelle
- 12: Schneidersche Membran
- 13: Kieferknochen
- 14: Stiftimplantat
- 15: Füllmaterial

## Patentansprüche

1. Implantat zum Überdecken von Knochendefekten, insbesondere im Kieferbereich, wobei das Implantat aus einer Magnesiumfolie besteht, wobei die Magnesiumfolie eine Dicke zwischen 70 und 200 µm aufweist, **dadurch gekennzeichnet, dass** die Magnesiumfolie zumindest in einem mittleren Bereich keine Öffnungen aufweist.

2. Implantat nach dem vorstehenden Anspruch, **dadurch gekennzeichnet, dass** das Implantat formstabil und gekrümmt ausgebildet ist, insbesondere mit einem Krümmungsradius von 0,5 bis 10 cm, bevorzugt von 0,7 bis 1,5 cm und/oder mit einem Winkel größer 30°.

3. Implantat nach einem der vorstehenden Ansprüche,
**dadurch gekennzeichnet, dass** das Implantat zumindest eine Aussparung für einen Zahn aufweist.

4. Implantat nach einem der vorstehenden Ansprüche,
**dadurch gekennzeichnet, dass** das Implantat eine geätzte Oberfläche aufweist.

5. Implantat nach einem der vorstehenden Ansprüche,
**dadurch gekennzeichnet, dass** das Implantat randseitig strukturiert und/oder perforiert ausgebildet ist.

6. Implantat nach einem der vorstehenden Ansprüche,
**dadurch gekennzeichnet, dass** die Magnesiumfolie weniger als 500 ppm Eisen, Kupfer und/oder Nickel aufweist.

7. Set zum Überdecken von Knochendefekten im Kieferbereich umfassend ein Implantat nach einem der vorstehenden Ansprüche, sowie Pins, Schrauben oder Fäden zur Fixierung des Implantats im Kiefer.

8. Verfahren zum Herstellen eines Implantats nach einem der vorstehenden Ansprüche, wobei eine Magnesiumfolie bereit gestellt wird, die Magnesiumfolie auf die Länge einer zu überdeckenden Stelle gekürzt wird und die Magnesiumfolie gebogen wird.

9. Verfahren nach dem vorstehenden Anspruch, **dadurch gekennzeichnet, dass** die Magnesiumfolie mit Flusssäure behandelt wird.

## Claims

1. An implant for covering bone defects, in particular in a jaw region, wherein the implant consists of a magnesium film, wherein the magnesium film has a thickness between 70 and 200 pm, **characterized in that** the magnesium film has no openings, at least in a central area thereof.

2. The implant as claimed in the preceding claim, wherein the implant is geometrically stable and has a curved shape, in particular with a radius of curvature from 0.5 to 10 cm, preferably from 0.7 to 1.5 cm, and/or with an angle of greater than 30°.

3. The implant as claimed in any of the preceding claims, wherein the implant has at least one recess for a tooth.

4. The implant as claimed in any of the preceding claims, wherein the implant has an etched surface.

5. The implant as claimed in any of the preceding claims, wherein the implant is structured and/or perforated along a peripheral area thereof.

6. The implant as claimed in any of the preceding claims, wherein the magnesium film contains less than 500 ppm of iron, copper, and/or nickel.

7. A kit for covering bone defects in a jaw region, comprising an implant as claimed in any of the preceding claims, and pins, screws, or sutures for fixing the implant in the jaw.

8. A method for producing an implant according to any of the preceding claims, comprising:
providing a magnesium film; shortening the magnesium film to a length of a site to be covered; and bending the magnesium film.

9. The method as claimed in the preceding claim, comprising treating the magnesium film with hydrofluoric acid.

## Revendications

1. Implant destiné à recouvrir des défauts osseux,
en particulier dans la région de la mâchoire, l'implant se composant d'un film de magnésium, le film de magnésium présentant une épaisseur comprise entre 70 et 200 um, **caractérisé en ce que** le film de magnésium, du moins dans une zone centrale, ne présente pas d'ouvertures.

2. Implant selon la revendication précédente, **caractérisé en ce que** l'implant est indéformable et de forme incurvée, en particulier avec un rayon de courbure de 0,5 à 10 cm, préférablement de 0,7 à 1,5 cm et/ou avec un angle supérieur à 30°.

3. Implant selon l'une des revendications précédentes, **caractérisé en ce que** l'implant présente au moins un évidement pour une dent.

4. Implant selon l'une des revendications précédentes, **caractérisé en ce que** l'implant présente une surface gravée.

5. Implant selon l'une des revendications précédentes, **caractérisé en ce que** l'implant côté bord est structuré et/ou perforé.

6. Implant selon l'une des revendications précédentes, **caractérisé en ce que** le film de magnésium contient moins de 500 ppm de fer, de cuivre et/ou de nickel.

7. Kit destiné à recouvrir des défauts osseux dans la région de la mâchoire, comprenant un implant selon l'une des revendications précédentes, ainsi que des broches, des vis ou des fils pour fixer l'implant dans la mâchoire.

8. Procédé de fabrication d'un implant selon l'une des revendications précédentes, un film de magnésium étant fourni, celui-ci étant raccourci à la longueur d'une zone à recouvrir et le film de magnésium étant plié.

9. Procédé selon la revendication précédente, **caractérisé en ce que** le film de magnésium est traité avec de l'acide fluorhydrique.
